# FASCICULE DE BREVET EUROPEEN

(11) **EP 1 073 373 B1**
(45) Date de publication et mention de la délivrance du brevet: **02.03.2005**
(21) Numéro de dépôt: 99914633.5
(22) Date de dépôt: 21.04.1999
(51) Int. Cl.: A61B 17/04

(54) **IMPLANT OSSEUX COMPRENANT UN DISPOSITIF DE FIXATION REVERSIBLE**
KNOCHENIMPLANTAT MIT EINER REVERSIBLEN BEFESTIGUNGSVORRICHTUNG
BONE IMPLANT WITH A REVERSIBLE FIXING DEVICE

(30) Priorité: 21.04.1998 FR 9805202
(43) Date de publication de la demande: 07.02.2001
(73) Titulaire: Tornier SA, 38330 Saint Ismier (FR)
(72) Inventeur: TORNIER, Alain, F-38330 Saint Ismier (FR); BONNOMET, François, F-67000 Strasbourg (FR)
(74) Mandataire: Schmitt, John
(86) Numéro de dépôt international: PCT/FR1999/000940
(87) Numéro de publication internationale: WO 1999/053843

(56) Documents cités:
- GB-A- 2 173 565
- US-A- 5 472 452
- US-A- 5 501 695
- US-A- 5 649 963

## Description

La présente invention est relative à un dispositif réversible pour la fixation d'un implant dans l'os d'un patient.

On connaît d'après le brevet américain n° 5 501 695 un dispositif de fixation dans l'os d'une ancre de suture qui est constitué de deux parties distinctes.

Le dispositif de fixation comprend un premier élément extérieur cylindrique solidaire de branches de fixation qui sont séparées les unes des autres par des fentes disposées parallèlement à l'axe longitudinal dudit dispositif. Le premier élément reçoit, dans sa partie interne, un second élément de déformation qui coopère avec l'extrémité libre des branches de fixation.

Le second élément de déformation irréversible est solidaire par l'intermédiaire d'une zone de rupture d'une tige de traction qui permet aux chirurgiens, après avoir introduit l'ancre de suture dans un trou préalablement ménagé dans l'os, de faire coulisser ledit second élément à l'intérieur du premier, afin de déformer axialement les branches de fixation dans la partie de l'os spongieux.

Lorsque l'effort de traction est suffisant pour déformer les branches de fixation, la tige se sépare du second élément par une rupture irréversible.

On note que les branches se déforment latéralement suivant une direction sensiblement perpendiculaire à l'axe longitudinal de l'ancre de suture pour fixer définitivement cette dernière à l'intérieur de l'os.

Enfin, le premier élément est solidaire à l'une de ses extrémités, d'une collerette qui vient en appui contre l'os cortical et qui est percée d'un certain nombre de trous pour la fixation par le chirurgien de fils de suture.

Le dispositif de fixation décrit ci-dessus comporte certains inconvénients, à savoir qu'il ne permet pas le retrait de l'ancre de suture de l'os sans provoquer une destruction complète de cette dernière et de l'os dans laquelle elle est fixée. En effet l'ancre de suture ne comporte aucun moyen de reprise permettant son retrait de l'os sans engager une détérioration de ce dernier.

En outre, le dispositif de fixation ne comporte pas en dehors de la zone de rupture du second élément, des moyens limitant la course dudit élément pour éviter que les branches de fixation viennent à se rompre anormalement sous l'effort de traction.

On connaît également, d'après le brevet US 5 472 452, un dispositif de fixation pour une ancre de suture comportant des moyens d'expansion qui sont déformés par l'intermédiaire d'un outil.

Ce dispositif de fixation, comporte des moyens d'expansion qui sont réversibles suivant le sens de l'effort de l'outil, et plus particulièrement de la constitution de l'outil dont le poussoir présente un pan incliné qui permet aux moyens d'expansion de descendre à l'intérieur du corps de l'ancre de suture pour pouvoir la retirer.

Ce dispositif de fixation concernant une ancre de suture ne comporte pas de moyens pour limiter la déformation plastique des moyens d'expansion lorsqu'un effort de traction est appliqué, afin d'éviter une rupture anormale, ou un dépassement de la déformation plastique rendant le dispositif de fixation non réversible.

On connaît d'autres dispositifs de fixation par ancrage en force ou par vissage qui ne permettent pas un retrait de l'implant sans occasionner une détérioration de l'os. Le document GB-A-2 173 565 décrit un implant, destiné à être fixé dans l'os d'un patient, comprenant les caractéristiques du préambule de la revendication 1.

Le dispositif de fixation de l'implant suivant la présente invention à pour objet d'être réversible, permettant ainsi son extraction de l'os sans avoir à percer un trou à un diamètre plus grand que celui des branches déformées.

Le dispositif de fixation de l'implant conforme à l'invention comprend des moyens d'expansion qui sont réversibles suivant le sens de l'effort afin de permettre soit la fixation de l'implant dans l'os, soit son retrait

Le dispositif de fixation de l'implant suivant la présente invention comprend des moyens d'expansion qui sont limités dans leur déformation plastique lors de l'application d'un effort permettant la fixation de l'implant dans l'os par des moyens de butée, afin que lesdits moyens d'expansion soient réversibles lors de l'application d'un autre effort permettant le retrait dudit implant de l'os.

Le dispositif de fixation de l'implant suivant la présente invention comporte des moyens d'expansion qui sont constitués d'une partie cylindrique percée d'un alésage interne fileté, d'au moins deux branches de fixation disposées parallèlement à l'axe longitudinal et dans le prolongement de la partie cylindrique, d'au moins deux butées intercalées entre chaque branche et limitant la déformation plastique de ces dernières, et, de préférence, d'une pointe à profil conique percée dans sa partie interne d'un trou borgne fileté.

Le dispositif de fixation de l'implant suivant un mode de réalisation préféré l'invention présente comporte des branches de fixation qui sont raccordées à la partie cylindrique et la pointe à profil conique par des premières amorces de pliage dirigées en direction du centre du dispositif de fixation.

Le dispositif de fixation de l'implant suivant un mode de réalisation préféré la présente invention comporte des branches de fixation qui présentent respectivement en leur milieu une amorce de pliage qui est inversée par rapport aux amorces de pliage de manière que chaque branche soit constituée de deux segments identiques.

Le dispositif de fixation de l'implant suivant un mode de réalisation préféré la présente invention comporte un alésage interne fileté et un trou borgne fileté qui sont portés par le même axe longitudinal et sont prévus de diamètres différents.

Le dispositif de fixation de l'implant suivant un mode de réalisation préféré la présente invention comporte des butées qui s'étendent parallèlement à l'axe longitudinal et présentent une longueur qui détermine la déformation plastique des branches de fixation.

Le dispositif de fixation de l'implant suivant un mode de réalisation préféré la présente invention comporte une pointe à profil conique qui est disposée perpendiculairement à l'axe longitudinal.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue illustrant un implant de forme quelconque muni du dispositif de fixation de l'implant suivant la présente invention.
Figure 2a à 2c sont des vues schématiques montrant la mise en place de l'implant dans un os au moyen du dispositif de fixation de l'implant suivant la présente invention.
Figure 3a à 3c sont des vues schématiques représentant l'extraction de l'implant de l'os du fait de la réversibilité du dispositif de fixation de l'implant suivant la présente invention.

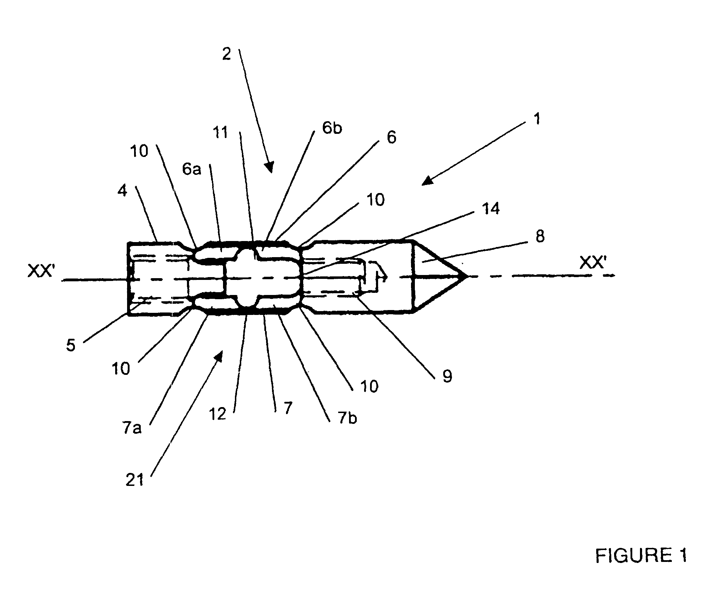

On a montré en figure 1 un implant 1 qui peut présenter une forme quelconque et sur lequel est prévu un dispositif de fixation 2 comportant des moyens d'expansion 21 qui sont réversibles pour permettre au chirurgien de pouvoir réintervenir sur l'implant 1 sans provoquer une détérioration trop importante de l'os 3.

Le dispositif de fixation 2 comporte une partie cylindrique creuse 4 qui est percée dans sa partie inteme d'un alésage fileté 5.

La partie cylindrique 4 du dispositif de fixation 2 se prolonge par au moins deux branches de fixation 6, 7 qui sont, avant déformation, parallèles à l'axe longitudinal XX' de ladite partie cylindrique 4.

Le dispositif de fixation 2, comporte dans le prolongement des branches 6 et 7, une pointe à profil conique 8 facilitant la mise en place de l'implant 1 dans l'os 3.

La partie cylindrique 4 présente un alésage interne fileté 5 porté par l'axe longitudinal XX' et qui débouche d'une part à l'extérieur de l'implant 1, et d'autre part entre les branches de fixation 6 et 7.

Également la pointe à profil conique 8 du dispositif de fixation 2 présente dans sa partie interne un trou borgne fileté 9 qui débouche entre les branches de fixation 6 et 7, et qui est porté par le même axe longitudinal XX' que celui de l'alésage 5.

De plus, le diamètre de l'alésage fileté 5 est prévu plus grand que celui du trou borgne fileté 9.

Les branches 6 et 7 sont raccordées à la partie cylindrique 4 et à la pointe 8 par des amorces de pliage 10 dirigées en direction du centre du dispositif de fixation 2 et qui permettront de déformer lesdites branches sous un effort de traction.

Les branches 6 et 7 présentent respectivement en leur milieu une amorce de pliage 11, 12 qui est inversée par rapport à celles 10 de manière que chaque branche soit constituée de deux segments 6a, 6b et 7a, 7b.

On note que les amorces de pliage 10, 11 et 12 présentent un profil en arc de cercle de rayon constant.

Entre chaque branche 6 et 7 est prévue une butée 13 solidaire de la partie cylindrique 4 et qui est dirigée en direction de la pointe à profil conique 8. Chaque butée 13 s'étend parallèlement à l'axe longitudinal XX' et présente une longueur qui dépend de la déformation que l'on désire obtenir des branches 6 et 7.

En effet la déformation des branches 6 et 7 est limitée par les butées 13 qui viennent en appui contre une face 14 de la pointe à profil conique 8. La face 14 est disposée dans un plan perpendiculaire à celui portant l'axe XX'.

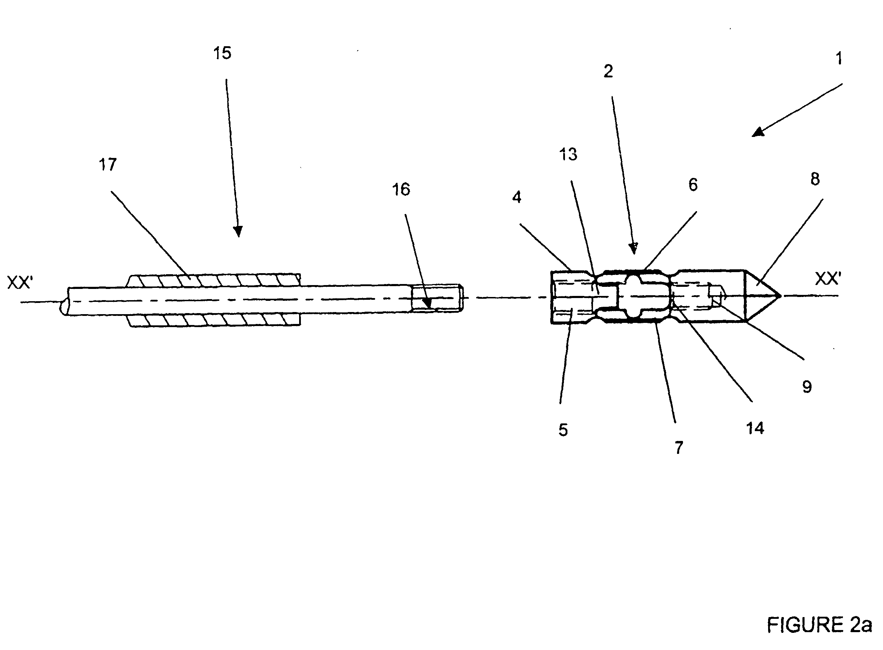

On a représenté en figures 2a à 2c les différentes étapes pour la mise en place de l'implant 1 pourvu du dispositif de fixation 2 à l'intérieur de l'os 3.

La figure 2a montre l'implant 1 solidaire d'un premier ancillaire 15 de mise en place qui est constitué, par exemple, d'une tige 16 qui traverse la partie interne du dispositif de fixation 2 pour venir se visser dans le trou borgne 9 de la pointe à profil conique 8. La tige 16 est solidaire d'un embout 17 qui vient prendre appui contre la partie cylindrique 4.

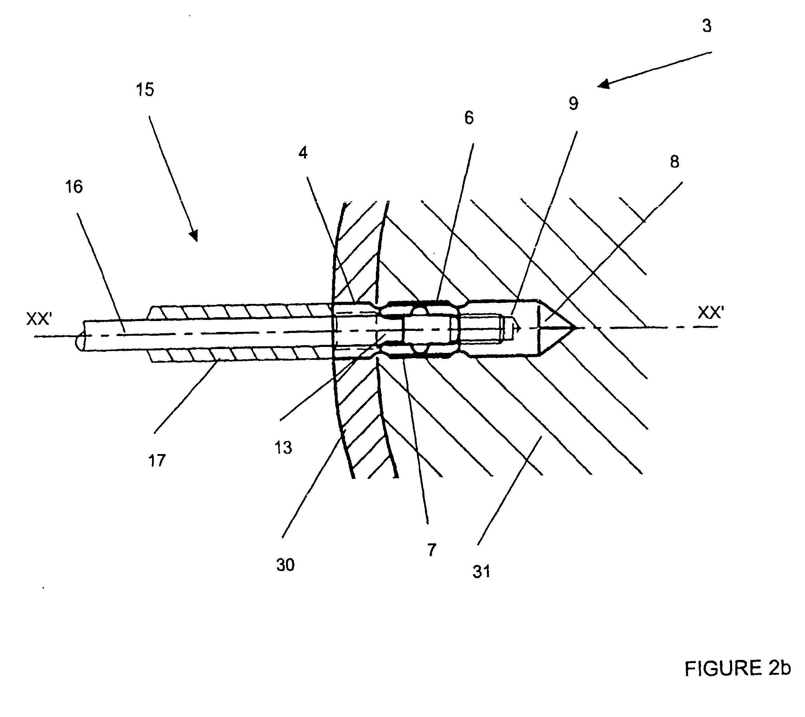

La figure 2b représente l'implant 1 qui est introduit dans le site opératoire par l'intermédiaire de l'ancillaire 15.

La mise en place de l'implant 1 dans l'os 3 est réalisée soit par force, soit par rotation, soit par l'intermédiaire d'un pré-trou percé dans l'os cortical 30 et l'os spongieux 31.

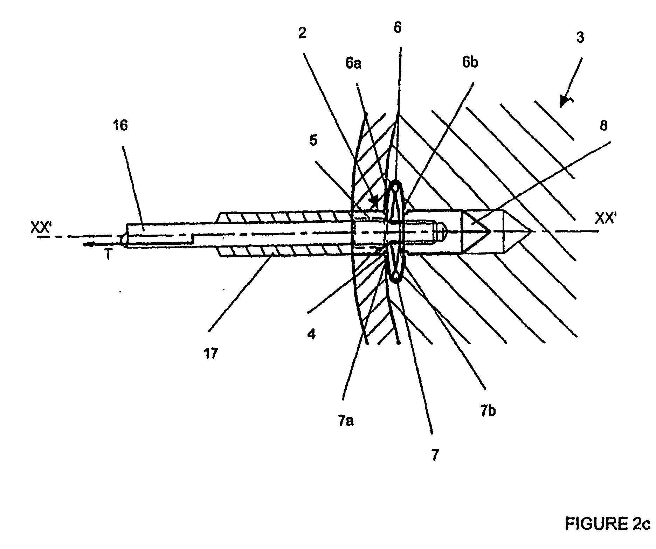

La figure 2c montre la déformation du dispositif de fixation 2 et plus particulièrement des branches 6 et 7 à l'intérieur de l'os spongieux 31 lorsqu'un effort de traction T est soumis à la tige 16 de l'ancillaire 15. Ainsi la tige 16 se déplace horizontalement suivant l'axe XX', tandis que l'embout 17 reste fixe en appui contre la partie cylindrique 4.

La déformation des branches 6 et 7 est limitée jusqu'à ce que la pointe à profil conique 8 vienne par l'intermédiaire de sa face 14 en appui contre les butées 13.

Les branches 6 et 7 se déforment, sous un effort de compression du fait de la traction T soumise à la tige 16 de l'ancillaire 15, suivant le profil des amorces 10, 11 et 12 de manière que les segments 6a, 6b et 7a, 7b soit dirigés à l'extérieur de l'implant 1 et dans une direction sensiblement perpendiculaire à l'axe XX'.

On note que la fixation de l'implant 1 dans l'os spongieux 31 est réalisée par la déformation des branches 6 et 7 jusqu'à ce que les segments 6a et 7a viennent en contact avec la face interne de l'os cortical 30.

On dévisse ensuite la tige 17 de l'ancillaire 16 pour libérer l'implant 1.

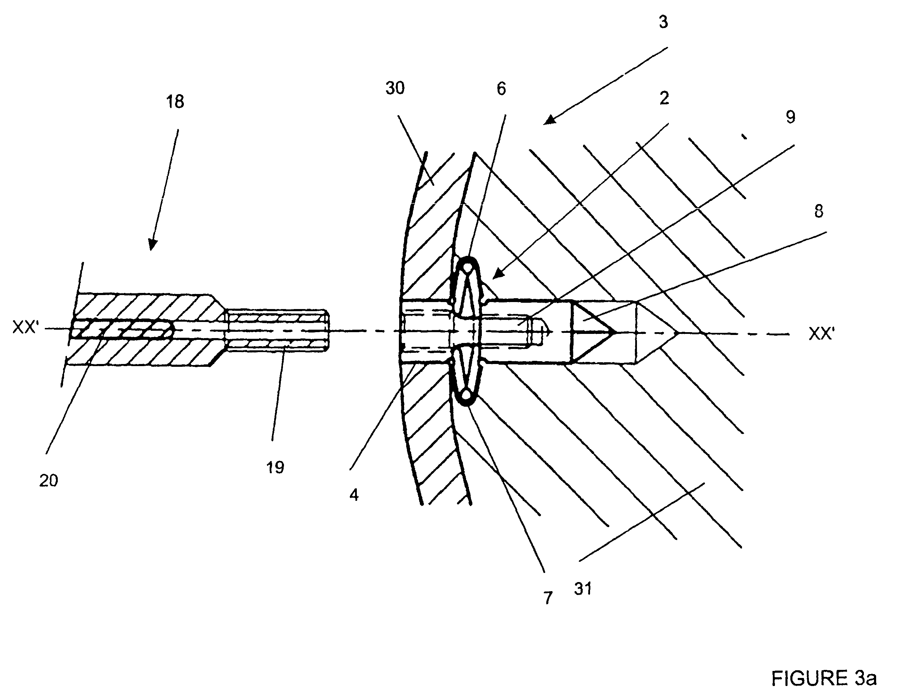

En figures 3a à 3c on a illustré les différentes étapes pour extraire l'implant 1 de l'os 3 au moyen d'un autre ancillaire 18 permettant la réversibilité du dispositif de fixation 2.

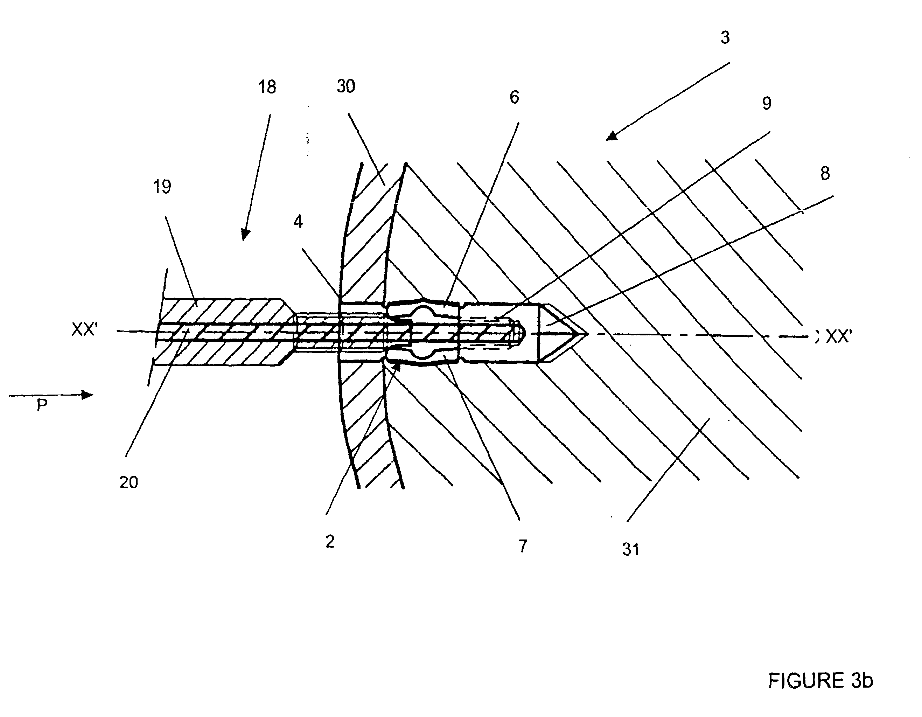

L'ancillaire 18 comporte une tige creuse 19 qui vient se visser dans l'alésage fileté 5 de la partie cylindrique 4, tandis qu'une autre tige 20 coulissant dans la première vient prendre appui dans le fond du trou borgne 9 ménagé dans la pointe à profil conique 8 (figure 3b).

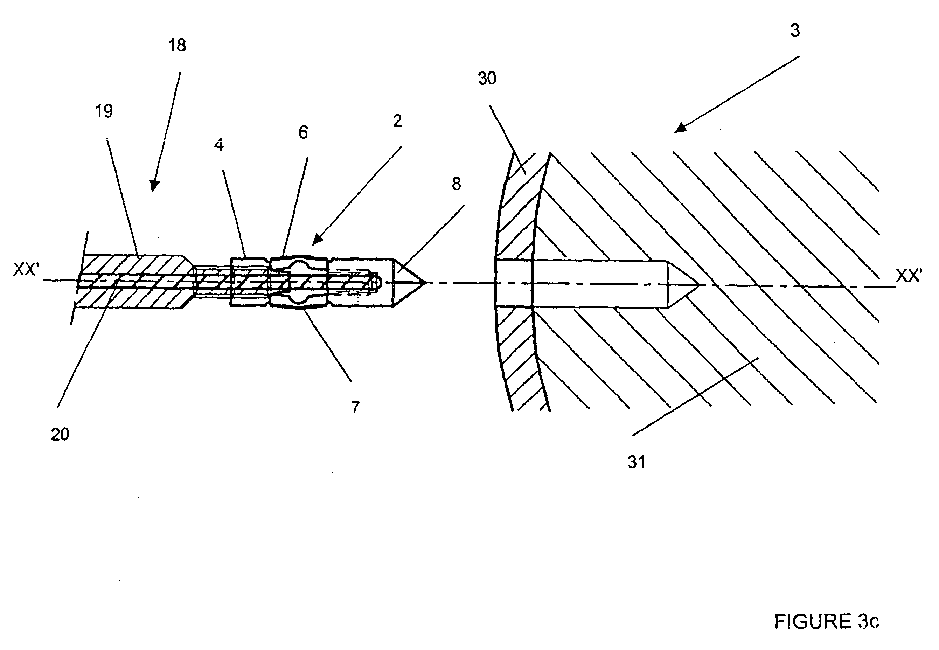

La tige 20 est soumise à un effort de poussée P parallèle à l'axe XX' afin de déplier les branches 6 et 7 (figure 3c). Le profil des amorces 10, 11 et 12 permet de ramener le dispositif de fixation 2 suivant une forme semblable à celle d'origine.

Dès que le dispositif de fixation 2 a retrouvé une position allongée, le chirurgien peut à l'aide de l'ancillaire 18 retirer l'implant 1 de l'os 3, sans à avoir à percer un trou dont le diamètre est sensiblement voisin de celui des branches déformées.

Le dispositif de fixation décrit précédemment a été appliqué comme exemple de réalisation à une ancre de suture. Bien évidemment, et sans pour autant changer l'objet de la présente invention, le dispositif de fixation réversible est destiné à être appliqué sur tout type d'implant prévu pour être fixé dans l'os d'un patient.

## Revendications

1. Implant chirurgical, destiné à être fixé dans l'os d'un patient, comprenant un dispositif de fixation réversible (2) comprenant des moyens d'expansion (21) qui sont constitués d'une partie cylindrique (4) percée d'un alésage interne fileté, (5), d'au moins deux branches de fixation (6, 7) disposées parallèlement à l'axe longitudinal de l'implant (XX') et dans le prolongement de la partie cylindrique (4) avant déformation plastique, **caractérisé en ce qu'**il comprend par ailleurs, au moins deux butées (13) intercalées entre chaque branche (6, 7) et limitant la déformation plastique de ces dernières lors de l'application d'un effort extérieur de traction (T) pour la fixation de l'implant (1) dans l'os (3) afin que la déformation desdits moyens d'expansion (21) soit réversible lors de l'application d'un autre effort extérieur de poussée (P) permettant le retrait de l'implant (1) de l'os (3).

2. Implant chirurgical suivant la revendication 1 **caractérisé en ce que** les moyens d'expansion (21) sont constitués dans le prolongement des branches de fixation (6, 7) d'une pointe à profil conique (8) percée dans sa partie interne d'un trou borgne fileté (9).

3. Implant chirurgical suivant la revendication 2 **caractérisé en ce que** les branches de fixation (6, 7) sont raccordées à la partie cylindrique (4) et à la pointe à profil conique (8) par des premières amorces de pliage (10) dirigées en direction du centre du dispositif de fixation (2).

4. Implant chirurgical suivant la revendication 3 **caractérisé en ce que** les branches de fixation (6, 7) présentent respectivement en leur milieu une seconde amorce de pliage (11, 12) qui est inversée par rapport aux premières amorces de pliage (10) de manière que chaque branche soit constituée de deux segments identiques (6a, 6b ; 7a, 7b).

5. Implant chirurgical suivant la revendication 2 **caractérisé en ce que** l'alésage interne fileté (5) et le trou borgne fileté (9) sont portés par l'axe longitudinal (XX') et sont prévus de diamètres différents.

6. Implant chirurgical suivant la revendication 2 **caractérisé en ce que** les butées (13) s'étendent parallèlement à l'axe longitudinal (XX') et présentent une longueur qui détermine la déformation plastique des branches de fixation (6, 7).

7. Implant chirurgical suivant la revendication 2 **caractérisé en ce que** les butées (13) viennent prendre appui contre une face (14) de la pointe à profil conique (8).

## Claims

1. Implant to be fixed in a bone of a patient, comprising a reversible fixation device (2) with expansion means (21) which are made up of a cylindrical part (4), in which a threaded internal bore (5) is provided, and of at least two fixation branches (6, 7) which are arranged parallel to the longitudinal axis (XX') of the implant and in the continuation of the cylindrical part (4) prior to plastic deformation, **characterized in that** it also comprises at least two stops (13) interposed between each branch (6, 7) and limiting the plastic deformation of these branches upon application of an external tractive force (T) for fixing the implant (1) in the bone (3), so that the deformation of said expansion means (21) is reversible upon application of an external thrust force (P) permitting removal of the implant (1) from the bone (3).

2. Surgical implant according to Claim 1, **characterized in that** the expansion means (21) are formed, in the continuation of the fixation branches (6, 7), by a conically profiled point (8) which is provided internally with a threaded blind hole (9).

3. Surgical implant according to Claim 2, **characterized in that** the fixation branches (6, 7) are joined to the cylindrical part (4) and to the conically profiled point (8) by first bend initiators (10) directed towards the centre of the fixation device (2).

4. Surgical implant according to Claim 3, **characterized in that** the fixation branches (6, 7) have, at their centre, a second bend initiator (11, 12) which is inverted in relation to the first bend initiators (10) so that each branch is made up of two identical segments (6a, 6b; 7a, 7b).

5. Surgical implant according to Claim 2, **characterized in that** the threaded internal bore (5) and the threaded blind hole (9) are carried by the longitudinal axis (XX') and are of different diameters.

6. Surgical implant according to Claim 2, **characterized in that** the stops (13) extend parallel to the longitudinal axis (XX') and have a length determining the plastic deformation of the fixation branches (6, 7).

7. Surgical implant according to Claim 2, **characterized in that** the stops (13) bear against a face (14) of the conically profiled point (8).

## Patentansprüche

1. Implantat, das in dem Knochen eines Patienten befestigt werden soll, mit einer Vorrichtung zur reversiblen Befestigung (2) mit Expansionsmitteln (21), die aus einem zylindrischen Teil (4), der von einer Gewindeinnenbohrung (5) durchdrungen wird, und zumindest zwei Befestigungsarmen (6, 7) bestehen, die vor der plastischen Verformung parallel zu der Längsachse des Implantats (XX') und in Verlängerung des zylindrischen Teils (4) angeordnet sind, **dadurch gekennzeichnet, dass** es unter anderem zumindest zwei Anschläge (13) umfasst, die zwischen jeden Arm (6, 7) eingefügt sind und die plastische Deformation dieser letzteren beim Anlegen einer äußeren Zugkraft (T) zur Befestigung des Implantats im Knochen (3) begrenzen, damit die Deformation der Expansionsmittel (21) beim Anlegen einer weiteren äußeren Schubkraft (P) reversibel ist und den Austritt des Implantats (1) aus dem Knochen (3) gestattet.

2. Chirurgisches Implantat nach Anspruch 1, **dadurch gekennzeichnet, dass** die Expansionsmittel (21) in der Verlängerung der Befestigungsarme (6, 7) aus einer Spitze mit konischem Profil (8) bestehen, die in ihrem inneren Teil von einem Gewindesackloch (9) durchbohrt ist.

3. Chirurgisches Implantat nach Anspruch 2, **dadurch gekennzeichnet, dass** die Befestigungsarme (6, 7) an das zylindrische Teil (4) und an die Spitze mit konischem Profil (8) durch erste Biegungsausgangspunkte (10) angeschlossen sind, die in Richtung der Mitte der Befestigungsvorrichtung (2) zeigen.

4. Chirurgisches Implantat nach Anspruch 3, **dadurch gekennzeichnet, dass** die Befestigungsarme (6, 7) jeweils in ihrer Mitte einen zweiten Biegungsausgangspunkt (11, 12) aufweisen, der relativ zu den ersten Biegungsausgangspunkten (10) verkehrt ist, so dass jeder Arm aus zwei identischen Segmenten (6a, 6b; 7a, 7b) besteht.

5. Chirurgisches Implantat nach Anspruch 2, **dadurch gekennzeichnet, dass** die Gewindeinnenbohrung (5) und das Gewindesackloch (9) durch die Längsachse (XX') getragen werden und mit unterschiedlichen Durchmessern versehen sind.

6. Chirurgisches Implantat nach Anspruch 2, **dadurch gekennzeichnet, dass** die Anschläge (13) sich parallel zur Längsachse (XX') erstrecken und eine Länge aufweisen, die die plastische Verformung der Befestigungsarme (6, 7) bestimmt.

7. Chirurgisches Implantat nach Anspruch 2, **dadurch gekennzeichnet, dass** die Anschläge (13) an einer Fläche (14) der Spitze mit konischem Profil (8) zur Anlage kommen.
